# FASCICULE DE BREVET EUROPEEN

(11) **EP 4 419 065 B1**
(45) Date de publication et mention de la délivrance du brevet: **03.12.2025**
(21) Numéro de dépôt: 22809035.3
(22) Date de dépôt: 21.10.2022
(51) Int. Cl.: A61K 8/34, A61L 9/00

(54) **COMPLEXE PARFUMANT, COMPOSITION PARFUMANTE ET COMPOSITION AQUEUSE PARFUMÉE D'ORIGINE NATURELLE**
DUFTKOMPLEX, DUFTZUSAMMENSETZUNG UND PARFÜMIERTE WÄSSRIGE ZUSAMMENSETZUNG NATÜRLICHEN URSPRUNGS
FRAGRANCING COMPLEX, FRAGRANCING COMPOSITION AND FRAGRANCED AQUEOUS COMPOSITION OF NATURAL ORIGIN

(30) Priorité: 22.10.2021 FR 2111249
(43) Date de publication de la demande: 28.08.2024
(73) Titulaire: Expressions Parfumees, 06130 Grasse (FR)
(72) Inventeur: MARIN, Christophe, 06200 NICE (FR); BUZZI, Jennifer, 06130 GRASSE (FR); SERY, Juliette, 06600 ANTIBES (FR)
(74) Mandataire: Hautier IP
(86) Numéro de dépôt international: PCT/EP2022/079455
(87) Numéro de publication internationale: WO 2023/067179

(56) Documents cités:
- EP-A1- 1 543 829
- EP-A1- 1 543 830
- EP-A1- 2 316 408
- WO-A1-2005/123028
- WO-A1-95/12379
- CN-A- 106 691 896
- US-B2- 7 854 940

## Description

### DOMAINE TECHNIQUE

La présente invention concerne le domaine des parfums et des compositions parfumées. Elle trouve pour application particulièrement avantageuse le domaine de la parfumerie fine, des cosmétiques, des produits d'hygiène à rincer ou non, et des parfums d'ambiance ou désodorisants d'intérieur, des détergents ou air fresheners.

### ETAT DE LA TECHNIQUE

Dans le domaine de la parfumerie, un problème récurrent concerne la solubilité des parfums dans l'eau.

Pour assurer à la fois une solubilité et une performance olfactive suffisantes à l'utilisateur, il s'est avéré nécessaire d'ajouter certains additifs. De tels additifs présentent notamment l'inconvénient de laisser un toucher gras et collant et pour la plupart d'irriter la peau. Ils modifient également les propriétés de la solution qui les contient comme la viscosité et la proportion de mousse formée. De nombreuses compositions actuellement commercialisées contiennent de l'éthanol. Or, l'éthanol est un COV (Composé Organique Volatil) ce qui est dommageable vis-à-vis de certaines contraintes réglementaires et environnementales. Parallèlement, des recherches ont permis de démontrer que la présence d'eau et la diminution voire l'absence de l'éthanol permettaient d'augmenter la ténacité du parfum dans le temps, d'éviter l'irritation et l'assèchement de la peau et des cheveux, de s'affranchir des risques liés à une exposition au soleil et d'éviter tout problème lié au stockage, transport (douanes) et à l'inflammabilité.

Afin de remédier aux inconvénients précités, il a été proposé des compositions à base de co-solvants tels que l'isosorbide, le solketal, et des éthers de ceux-ci. Il a également été proposé des compositions comprenant de l'alcool oléylique éthoxylé (commercialisé sous le nom d'Ameroscol^{®}) ou de sulfates, tels que le sodium lauryl sulfate et le sodium laureth sulfate, en tant que tensioactifs. Cependant, de tels composés sont irritants et asséchants pour la peau et les cheveux et sont donc à éviter dans le domaine de la parfumerie et de la cosmétique.

Une solution identifiée par la demanderesse est décrite dans la demande de brevet WO2019154892A1. Cette solution consiste en une composition comprenant des solvants choisis pour assurer un haut dosage de parfum sans les inconvénients cités ci-dessus. Cependant, la demande du consommateur est de plus en plus tournée vers des compositions d'origine naturelle. Les compositions parfumées proposées doivent toutefois permettre de solubiliser les huiles essentielles qui sont des composants très difficiles à solubiliser dans l'eau. De plus, la faible performance des solvants et solubilisants d'origine naturelle limitent fortement le dosage de parfum possible.

Par ailleurs, cela pose également la problématique de la transparence des compositions pour faciliter notamment leur usage dans divers domaines sans constituer une limitation. WO2005123028 divulgue une composition aqueuse sans éthanol, transparente et parfumée, sous forme de microémulsion, utilisable comme composition cosmétique, par exemple un spray pour le corps et un déodorant, comprenant une substance parfumée, un solvant tel qu'un diol vicinal, en particulier le 1,2-hexanediol, le 1,2-heptanediol et le 1,2-octanediol, un milieu aqueux et un agent tensioactif. EP2316408A1 divulgue une microémulsion d'huile parfumée sans éthanol, utile dans un article comprenant une eau de toilette et un désodorisant, comprenant de l'eau, un ou plusieurs diols vicinaux, par exemple le 1,2-pentanediol et le 1,2-octanediol, un ou plusieurs solvants, par exemple le glycérol, un ou plusieurs parfums et éventuellement d'autres substances.

En parfumerie, l'obtention d'un mélange odorant qui soit à la fois polyvalent en termes d'applications, transparent et qui présente des propriétés sensorielles améliorées, préférentiellement stable et d'origine naturelle, fait l'objet de recherches permanentes.

Il existe donc le besoin de proposer une composition parfumée qui résolve tout ou partie de ces inconvénients.

### RESUME DE L'INVENTION

Pour atteindre cet objectif, selon un aspect, l'invention concerne une composition parfumante comprenant les composants suivants :
- de 25 à 34% du complexe parfumant tel que décrit ci-dessous,
- de 66 à 75% d'un mélange de Polyglyceryl-6 Caprylate/Caprate et Sodium Caproyl/Lauroyl Lactylate,
les pourcentages étant exprimés en poids par rapport au poids total des 2 composants ci-dessus.

Le complexe parfumant comprenant les composants suivants :
- 5 à 70% d'un parfum,
- 10 à 30% de caprylyl glycol comme solvant, et
- 0 à 85% de mono propylène glycol comme solvant,
les pourcentages étant exprimés en poids par rapport au poids total des 3 composants ci-dessus.

Ce complexe parfumant présente l'avantage de permettre la solvatation d'un parfum à haut dosage dans des solvants dit d'origine naturelle et qui ne sont pas habituellement utilisés pour cet usage et dans ces proportions. Notamment, le caprylyl glycol est habituellement connu comme un optimisateur des conservateurs utilisés pour la protection contre les micro-organismes. A cet effet, il est utilisé en faible quantité et son usage dans les proportions du présent complexe ne coule pas d'une démarche évidente en ce qu'il est plutôt recherché une quantité minimale pour un effet optimal pour ce type de produit.

Avantageusement, le complexe parfumant ne comprend pas d'eau.

Le complexe parfumant selon l'invention présente l'avantage de ne pas contenir d'eau, ce qui facilite son transport, son conditionnement tout en étant stable et non irritant pour la peau.

La composition parfumante présente l'avantage d'utiliser un solubilisant également connu sous le nom d'Easytens S2 qui est d'origine naturelle et qui présente une synergie de solubilisation avec les solvants du parfum et en particulier avec le mono propylène glycol.

Avantageusement, la composition parfumante ne contient pas d'eau. On entend par là que la composition parfumante ne contient pas d'eau ajoutée en plus des composants de ladite composition. Comme pour le complexe parfumant, cela facilite son transport, son conditionnement tout en étant stable et non irritant pour la peau.

Pour atteindre cet objectif, selon un autre aspect, l'invention concerne une composition aqueuse parfumée comprenant les composants suivants :
- de 20 à 30% de la composition parfumante telle que décrite ci-dessus,
- de 70 à 80% d'eau,
les pourcentages étant exprimés en poids par rapport au poids total des 2 composants ci-dessus.

Avantageusement, la composition aqueuse parfumée est sous forme de micro-émulsion.

Suivant un autre aspect, l'invention concerne un procédé de préparation d'une composition parfumante telle que décrite ci-dessus :
- la préparation d'un complexe parfumant tel que décrit ci-dessus,
- le chauffage du mélange de Polyglyceryl-6 Caprylate/Caprate et Sodium Caproyl/Lauroyl Lactylate, préférentiellement à maximum 65°C,
- l'ajout du mélange de Polyglyceryl-6 Caprylate/Caprate et Sodium Caproyl/Lauroyl Lactylate chauffé au complexe parfumant,
- l'homogénéisation de la composition parfumante obtenue.

Suivant un autre aspect, l'invention concerne un procédé de préparation d'une composition aqueuse parfumée telle que décrite ci-dessus comprenant :
- l'ajout d'eau à la composition parfumante décrite ci-dessus, et
- l'homogénéisation de la composition aqueuse parfumée obtenue.

L'invention concerne également une base cosmétique, une base détergente ou un parfum d'ambiance comprenant la composition aqueuse parfumée ou la composition parfumante ou le complexe parfumant tels que décrits ci-dessus.

L'invention concerne également l'utilisation de la composition aqueuse parfumée, de la base cosmétique ou de la base détergente ou du parfum d'ambiance tels que décrits ci-dessus pour masquer les mauvaises odeurs.

Préférentiellement, le complexe parfumant, la composition parfumante, et la composition parfumée aqueuse sont d'origine naturelle, encore plus préférentiellement certifiable COSMOS. Enfin la formule élaborée est compatible, quel que soit le parfum et la famille olfactive choisis.

### DESCRIPTION DÉTAILLÉE

Avant d'entamer une revue détaillée de modes de réalisation de l'invention, sont énoncées ci-après des caractéristiques optionnelles qui peuvent éventuellement être utilisées en association ou alternativement.

Selon un exemple, la composition aqueuse parfumée ou la composition parfumante ou le complexe parfumant est transparent.

Selon un exemple, la composition aqueuse parfumée est sous forme de micro-émulsion.

Selon un exemple, la composition aqueuse parfumée ou la composition parfumante ou le complexe parfumant ne comprend pas de solvant éthoxylé.

Selon un exemple, le procédé de préparation de la composition aqueuse parfumée comprend une étape ultérieure d'ajout d'un conservateur, par exemple en quantité comprise entre 0.8% et 1.2%, préférentiellement en quantité inférieure ou égale à 1% en poids du poids total de la composition. Préférentiellement, le procédé comprend un mélange jusqu'à homogénéisation. Ce conservateur est un additif. De manière habituelle, les additifs et notamment les conservateurs sont comptés dans la quantité d'eau. Préférentiellement, la quantité d'eau décrite comprend l'eau et les éventuels additifs notamment de type conservateur.

Selon un exemple, le procédé de préparation de la composition aqueuse parfumée comprend une étape ultérieure de macération, de glaçage, préférentiellement à 4°, puis de filtration.

Selon un autre aspect, l'invention concerne l'utilisation de la composition aqueuse parfumée ou la composition parfumante ou le complexe parfumant tels que décrits ci-dessus pour la parfumerie fine.

Selon un autre aspect, l'invention concerne l'utilisation de la composition aqueuse parfumée ou la composition parfumante ou le complexe parfumant tels que décrits ci-dessus pour la cosmétique.

Selon un autre aspect, l'invention concerne l'utilisation de la composition aqueuse parfumée ou la composition parfumante ou le complexe parfumant tels que décrits ci-dessus pour les produits d'hygiène destinés à être rincés tels que des gels douche, des shampoings etc, ou non tels que des déodorants etc.

Selon un autre aspect, l'invention concerne l'utilisation de la composition aqueuse ou la composition parfumante ou le complexe parfumant tels que décrits ci-dessus pour des parfums d'ambiance ou désodorisants d'intérieur ou air fresheners ou produits de détergence.

Par « compatible avec n'importe quel type de famille olfactive », on entend que ladite composition peut contenir n'importe quel type de parfum, de n'importe quelle famille olfactive.

Par « haut dosage de parfum », on entend que la composition aqueuse parfumée peut contenir jusqu'à 10% en poids de complexe parfumant.

Par « stable » on entend une composition qui ne se dégrade pas avec le temps et dont les composés ne vont pas réagir entre eux. Par «stable», on entend que l'homogénéité, la coloration, l'intensité olfactive, la qualité olfactive et la viscosité de la composition sont conservées. Plus particulièrement, la composition selon la présente invention est stable en vieillissement accéléré après 24h aux UV (Suntest) et après 2 mois à 25°C et 45°C en comparaison avec un échantillon resté à 5°C, à l'abri de la lumière.

Par « sans-éthanol » ou "sans isopropanol", on entend une composition contenant 0% en poids d'alcool éthylique ou 0% en poids d'alcool isopropylique.

Par « sans composé éthoxylé », on entend une composition contenant 0% en poids de composés éthoxylés, c'est-à-dire contenant le motif -(-CH2-CH2-O-).

Par « transparente », on entend que ladite composition est transparente pour des températures comprises entre 4 et 50°C quel que soit le parfum utilisé. Par composition transparente, on entend une composition dont la turbidité est inférieure à 12 NTU (Nephelometric Turbidity Unit), de préférence inférieure à 7 NTU. Celle-ci étant préférentiellement mesurée à l'aide d'un turbidimètre de type HI88713 Iso Turbidimeter de la marque HANNA Instruments.

Par « odeur agréable », on entend une odeur qui est détectée par le sens olfactif de l'être humain et qui est perçue comme agréable.

Par « présentant une viscosité proche de celle de l'eau » on entend que la viscosité dynamique à 20°C de ladite composition est comprise entre 1 et 50 mPa.s, préférentiellement entre 1 et 40 mPa.s, préférentiellement entre 1 et 30 mPa·s, préférentiellement entre 1 et 10mPa.s. Selon la présente invention, la viscosité dynamique est mesurée avec le viscosimètre Viscotester IQ, géométrie CC27 DG/TI - 01160025, à 20°C, à 500 s-1 pendant 30 secondes.

Par « parfum », on entend une composition comprenant un mélange de substances parfumantes, lesdites substances parfumantes étant à l'état isolé, en solution ou en suspension, dans leurs diluants, dissolvants ou co-ingrédients habituels. Une telle composition est destinée à apporter une composante olfactive agréable.

Par « substance parfumante », on entend une ou plusieurs matières premières d'origine naturelle ou synthétiques parfumées.

Par « composition aqueuse parfumée », on entend une composition comprenant plus de 50% d'eau et un parfum ou un complexe parfumant ou une composition parfumante.

On entend par certifiable COSMOS que le complexe, la composition parfumante et/ou la composition aqueuse parfumée satisfait les critères pour obtenir le label COSMOS en vigueur en 2022.

Les pourcentages s'entendent en pourcentage massique c'est-à-dire en poids par rapport à un poids du complexe ou d'une composition.

On entend par base cosmétique des eaux de toilettes adultes, enfants, bébé, animaux, brume pour le corps, spray pour les cheveux, eau solaire, solutions pour lingettes etc.

On entend par base détergente un parfum pour le linge, un parfum pour lessive, un parfum pour le sol, etc.

On entend par parfum d'ambiance, une composition parfumée pour la maison, la voiture, les espaces clos publics, professionnels, privés ou environnementaux, les tissus, etc.

Selon un premier aspect, la présente invention concerne un complexe parfumant comprenant un parfum, un premier solvant et un deuxième solvant.

Le complexe parfumant comprend avantageusement un parfum en quantité comprise entre 5 et 70% en poids du poids total du complexe parfumant, préférentiellement de 5 à 55%, préférentiellement 5 à 50%. Le poids total du complexe parfumant s'entend comme la somme d'un parfum, du premier solvant et du deuxième solvant.

Le complexe parfumant comprend avantageusement un premier solvant en quantité comprise entre 0 et 85% en poids du poids total du complexe parfumant, préférentiellement de 35 à 85%. Préférentiellement, le premier solvant est le monopropylène glycol dont le numéro CAS est 57-55-6.

Le monopropylène glycol est un solvant hydrophile végétal certifié COSMOS utilisé en parfumerie. Sa formule chimique est la suivante :

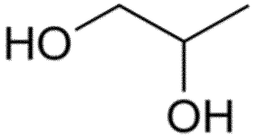

Son log P de -0.92 lui donne un caractère hydrophile particulièrement avantageux pour la formulation d'une micro-émulsion aqueuse. Néanmoins, c'est ce caractère hydrophile qui fait de lui un solvant de parfumerie peu efficace.

Le complexe parfumant comprend avantageusement un deuxième solvant en quantité comprise entre 10 et 30% en poids du poids total du complexe parfumant, éventuellement de 10 à 20%, voire de 10 à 15%.

Préférentiellement, le deuxième solvant est le caprylyl glycol. Le caprylyl glycol est connu sous le numéro CAS 1117-86-8. Le caprylyl glycol est également connu sous le nom de A-leen 8 ou 1,2 octanediol. Sa formule chimique est la suivante :

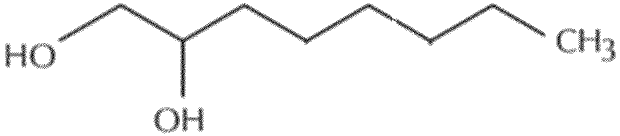

De façon surprenante, la demanderesse a identifié des propriétés de solvant à cette matière première connue pour ses propriétés d'amélioration des propriétés des conservateurs. Le caprylyl glycol permet de préparer une composition parfumée homogène et transparente.

Avantageusement, le complexe parfumant ne comprend pas d'eau.

Selon un mode de réalisation, le complexe parfumant est destiné à être utilisé dans une composition parfumante, préférentiellement ne contenant pas d'eau ajoutée ou une composition aqueuse parfumée c'est-à-dire mélangée à de l'eau.

Avantageusement, le complexe parfumant est constitué de 5 à 70% de parfum, de 0 à 85% de monopropylène glycol, de 10 à 30% de caprylyl glycol en poids du poids total du complexe parfumant.

Selon un deuxième aspect, la présente invention concerne une composition parfumante comprenant le complexe parfumant décrit ci-dessus et un solubilisant.

Préférentiellement, la composition parfumante comprend le complexe parfumant en quantité comprise entre 25 et 34% en poids du poids total de la composition parfumante. Le poids total de la composition parfumante s'entend comme la somme du complexe parfumant et du solubilisant.

Préférentiellement, la composition parfumante comprend le solubilisant en quantité supérieure ou égale à 66%, préférentiellement comprise entre 66 à 75% en poids du poids total de la composition parfumante. Selon un mode de réalisation préférée le solubilisant est un mélange de Polyglyceryl-6 Caprylate/Caprate (et) Sodium Caproyl/Lauroyl Lactylate connu également sous le nom de Easytens S2 commercialisé par Lavollée et produit par Hydrior.

Ce solubilisant est un mélange de tensioactifs COSMOS. Les propriétés solubilisantes des molécules contenues dans ce mélange ont été étudiées pour obtenir une synergie. Ce solubilisant permet d'obtenir des résultats très satisfaisants en coupage avec du parfum. Par ailleurs, de façon surprenante, ce solubilisant fonctionne en synergie avec les solvants sélectionnés pour le complexe parfumant, ce qui permet d'obtenir une solution transparente et homogène dans l'eau.Cette matière première est appropriée comme agent solubilisant dans une micro-émulsion COSMOS. Avantageusement, la composition parfumante est configurée pour ne pas mousser exagérément, ni présenter une viscosité trop élevée qui sont des paramètres qui rendent difficiles la mise en œuvre de la composition parfumante lors de la fabrication et/ou de la fabrication de la composition aqueuse parfumée.

Préférentiellement, la composition parfumante ne comprend pas d'eau ajoutée. La composition parfumante ne contient pas d'eau comme composant en plus des autres composants de la composition parfumante.

Préférentiellement, la composition parfumante est constituée de 25 à 34% du complexe parfumant en poids du poids total de la composition parfumante et d'au moins 66%, préférentiellement de 66 à 75% du mélange de Polyglyceryl-6 Caprylate/Caprate (et) Sodium Caproyl/Lauroyl Lactylate, en poids du poids total de la composition parfumante.

La composition parfumante comprend ainsi :
- de 1,25 à 23.8% de parfum,
- de 0 à 28.9% de Monopropylène glycol,
- de 2.5 à 10.2% de caprylyl glycol,
- de 66 à 75% de mélange de Polyglyceryl-6 Caprylate/Caprate (et) Sodium Caproyl/Lauroyl Lactylate.

Selon un troisième aspect, l'invention concerne une composition aqueuse parfumée comprenant la composition parfumante citée ci-dessus et de l'eau.

Avantageusement, la composition aqueuse parfumée comprend de 20 à 30% de la composition parfumante décrite ci-dessus. Préférentiellement, la composition aqueuse parfumée comprend la quantité restante en eau préférentiellement de 70 à 80% d'eau. Les pourcentages s'entendent en poids du poids total de la composition aqueuse parfumée. Préférentiellement, la quantité d'eau inclut les éventuels additifs tels que les conservateurs.

Avantageusement, la composition aqueuse parfumée comprend :
- de 5 à 10% du complexe parfumant décrit ci-dessus,
- de 13,2 à 22,5% du solubilisant, c'est-à-dire le mélange de Polyglyceryl-6 Caprylate/Caprate (et) Sodium Caproyl/Lauroyl Lactylate décrit ci-dessus, préférentiellement au moins 13,2%,
- et une quantité complémentaire d'eau pour atteindre 100%, c'est-à-dire classiquement de l'ordre de 70 à 80%.

Les pourcentages s'entendent en poids du poids total de la composition aqueuse parfumée.

La composition aqueuse parfumée comprend ainsi :
- de 0,25 à 7.14% de parfum,
- de 0 à 8.67% de Monopropylène glycol,
- de 0,5 à 3.06% de caprylyl glycol,
- de 13.2 à 22.5% de mélange de Polyglyceryl-6 Caprylate/Caprate (et) Sodium Caproyl/Lauroyl Lactylate, préférentiellement au moins 13,2%,
- de 70 à 80% d'eau.

Selon un mode de réalisation, la composition aqueuse parfumée comprend un conservateur. Préférentiellement, le conservateur est présent en quantité comprise entre 0,8 à 1,2%, préférentiellement inférieure ou égale à 1%. Avantageusement, la quantité de conservateur est prévue dans la quantité totale d'eau.

Avantageusement, la composition aqueuse parfumée et/ou la composition parfumante est transparente.

Avantageusement, la composition aqueuse parfumée est une micro-émulsion.

La composition aqueuse parfumée est dite aqueuse, car la composition comprend une majorité d'eau. Avantageusement, la composition aqueuse parfumée est une micro-émulsion huile dans eau.

Avantageusement, la composition aqueuse parfumée et/ou la composition parfumante ne comprend pas de solvant éthoxylé.

Avantageusement, la composition aqueuse parfumée et/ou la composition parfumante ne contient pas de parabène.

Avantageusement la composition aqueuse parfumée et/ou la composition parfumante ne contient pas de sulfate.

Avantageusement, la composition aqueuse parfumée présente une viscosité proche de l'eau. La texture est ainsi légère.

Avantageusement, la composition aqueuse parfumée est sprayable.

Avantageusement, la composition aqueuse parfumée et/ou la composition parfumante n'assèche pas la peau et présente ainsi des propriétés émollientes.

Avantageusement, la composition aqueuse parfumée mousse peu.

Avantageusement, la composition aqueuse parfumée et/ou la composition parfumante est d'origine naturelle.

Avantageusement, la composition aqueuse parfumée et/ou la composition parfumante est certifiable COSMOS.

Selon un autre aspect, l'invention concerne un procédé de préparation de la composition aqueuse parfumée.

Le procédé comprend la préparation du complexe parfumant. La préparation du complexe parfumant comprend le mélange du parfum avec le premier solvant et le deuxième solvant.

Le procédé comprend ensuite la préparation de la composition parfumante. Cette étape comprend un chauffage du mélange de Polyglyceryl-6 Caprylate/Caprate (et) Sodium Caproyl/Lauroyl Lactylate. Le chauffage est réalisé par les moyens connus de l'homme du métier tel que par exemple chauffage par convection par stockage en chambre chaude, ou chauffage par conduction (bain marie, bain de sable, plaque chauffage, double enveloppe, etc). Le chauffage est réalisé jusqu'à obtenir une viscosité permettant une facilité de mise en œuvre. Avantageusement, le chauffage est réalisé à une température de maximum 65°C.

L'étape de préparation de la composition parfumante comprend après l'étape de chauffage une étape de mélange du mélange de Polyglyceryl-6 Caprylate/Caprate (et) Sodium Caproyl/Lauroyl Lactylate chauffé avec le complexe parfumant. Le mélange est réalisé jusqu'à obtenir une composition parfumante homogène et transparente, avantageusement sans volute.

Le procédé comprend ensuite la préparation de la composition aqueuse parfumée à partir de la composition parfumante obtenue précédemment. La composition parfumante est mélangée avec de l'eau. Le mélange est réalisé jusqu'à obtenir une composition aqueuse parfumée homogène et transparente avantageusement sans volute.

Selon un mode de réalisation, le procédé comprend une étape d'ajout d'un conservateur à la composition aqueuse parfumée.

Selon un mode de réalisation, le procédé de fabrication la composition aqueuse parfumée comprend éventuellement une étape de macération, préférentiellement de 2 à 4 semaines, puis une étape de glaçage préférentiellement à 4°C et une étape de filtration. Cette étape est réalisée après l'ajout d'eau et du conservateur.

La composition parfumée aqueuse est apte à être conditionnée dans de nombreux types de packaging apte à recevoir une composition liquide aqueuse. Par exemple non limitatif des tubes, des flacons, des sprays, des vaporisateurs, des roll-on, des pots, des pinceaux à réservoir, des crayons à réservoir, etc.

Le parfum selon l'invention peut comprendre des ingrédients d'origine préférentiellement naturelle, éventuellement synthétique. Le choix de ce parfum dépend d'une part de l'effet odorant recherché et d'autre part l'application dans laquelle il va être intégré.

De telles matières premières, qu'elles soient d'origine naturelle ou synthétiques, peuvent comprendre des esters, éthers, alcools, aldéhydes, cétones, lactones, acétals, nitriles, phénols, acides, terpènes, composés hétérocycliques azotés ou soufrés, saturés ou insaturés, et des produits complexes d'origine naturelle.

Des exemples d'esters comprennent, mais ne sont pas limités à, l'acétate de benzyle, acétate de p-tert-butylcyclohexyle, 3,7-diméthyl-1,6-octadien-3-yl acetate (acétate de linalyle), acétate de diméthyl-benzyl-carbinyle, acétate de phényléthyle, acétate de 1,1-diméthyl-2-phényléthyle, benzoate de linalyle, glycinate d'éthyl-méthyl-phényle, propionate d'allylcyclohexyle, propionate de styrallyle, salicylate de benzyle, acétate de méthyl-3-oxo-2-pentylcyclopentane, acétate de prop-2-ènyle-2,3-méthylbutoxy (glycolate d'allyl amyle, ester 2-propénylique de l'acide 3-méthylbutoxy-acétique), ester phénylméthylique de l'acide acétique, acétate d'isoamyle (acétate d'isopentyle), acétate de cis-hex-3-ènyle (acétate de (Z)-hex-3-ènyle), acétate de citronellyle (acétate de 3,7-dimethyl-6-octèn-1-ol), acétate d'hexyle, acétate d'isobornyle (exo-acétate de bicyclo[2.2.1]heptan-2-ol,1,7,7-triméthyle), acétate de méthanyle (acétate d'alpha,alpha,4-triméthylcyclohexylméthyle), acétate d'éthyle, acétate de prényle (acétate de 3-méthyl-2-butényle), citrate de triéthyle, acétate de 4-ter-Butylcyclohexyle, acétate de (3R-(3alpha,3abeta,6alpha,7beta,8aalpha))-Octahydro-3,6,8,8-tetraméthyl-1H-3a-7-méthanoazulèen-5-yl, acétate de 3,7-Diméthyl octa-1,6-diène-3-yl, 1,4-Dioxacyclohexadecane-5,16-Dione, 2-hydroxybenzoate de benzyle, (Z)-3-Hexenyl-2-hydroxybenzoate, acétate de 2-(1,1(Diméthyléthyl) Cyclohexyle, acétate d'isopentyl, Méthyl Phénylacétate, acétate de (Z)-Hex-3-enyl, acétate de 3,7-Diméthyl octa-1,6-diene-3-yl, acétate de 3-Méthyl-2-butényl, acétate de alpha-Méthyle-Benzèneméthanol, 2-aminobenzoate de méthyle, 2-Propényl-(cyclohexyloxy)acétate, 2,4-dihydroxy-3,6-diméthylbenzoate de méthyle, 3-oxo-2-pentylcyclopentaneacétate de méthyle, Propanoate de 3-alpha,4,5,6,7,7-alpha-Hexahydro-4,7-methano-1H-inden-6-yl, méthyl 3-oxo-2-pentylcyclopentaneacétate, Acétate 2-(1,1-Diméthyléthyl)Cyclohexyl, hexyl-2-hydroxybenzoate (2-hydroxy-2-hexyl ester d'acide benzoïque), acétate de 3a,4,5,6,7,7a-Hexahydro-4,7-méthanoinden-6-yl, 2-méthylbutyrate d'éthyle, 3a,4,5,6,7,7a-Hexahydro-4,7-méthano-1H-inden-5-yl, 2-méthylpropanoate, Acétate de alpha,alpha-Diméthylphénéthyl, Acétate de 3,7-Diméthyl octa-1,6-diene-3-yl, Acétate d'exo-1,7,7-triméthyl-bicyclo[2.2.1]heptan-2-ol, hexanoate d'éthyle, acétate de 3,7-Diméthyl octa-1,6-diene-3-yl, méthyl 3-oxo-2-pentylcyclopentaneacétate, Méthyl 2-(méthylamino)benzoate, Acide 10-Undécenoïque, ester d'éthyle, 2-méthylpentanoate d'éthyle, éthanoate de cis-3,7-Diméthyl-2,6-octadienyle, 2-hydroxybenzoate de benzyle, acétate de (Z)-Hex-3-enyle, 2-hydroxy-2-hexyl ester d'acide benzoïque, prop-2-ènyl-2-cyclohexyloxyacetate (2-Propényl-(cyclohexyloxy)acétate), Acide acétique, (3-Methylbutoxy), Ester de 2-Propényle, 2-Phényléthanol, Acétate d'hexyle, (1R, 2S, 5R)-5-Méthyl-2-(1-méthyléthyl)-cyclohexanol éthanoate, acétate de terpényle (acétate de 4-méthyl-1-propan-2-yl-1-cyclohex-2-ènyle), formiate d'alpha-3,3-triméthylcyclohexyle-méthyle, butanoate de 3-méthylbutyle (butyrate d'iso amyle), butanoate d'alpha,alpha-diméthylphenethyl, acétate de 3-dihydrodicyclopentadièn-2,3-yle, prop-2-ènyl, propanoate de 3-cyclohexyle (cyclo hexane propionate d'allyl), heptanoate d'allyle (heptanoate de 2-propényl), 2-methylpropanoate de 2-phénoxy-éthyle (isobutyrate de phénoxy éthyle), 2-méthyl-pentanoate d'éthyle, 2-méthyl-butyrate d'éthyle (ester éthylique de l'acide 2-méthyl-butanoïque), 1,4-dioxacycloheptadecane-5,17-dione (Brassylate d'éthylène), propanoate de (2S)-2-propyl-1,1-dimethyl¬propoxy ((2S)-Ester propylique de l'acide 2-(1,1-diméthylpropoxy)-propanoïque), acétate de 2-tert-butylcyclohexyle (acétate de 2-(1,1-diméthylethyl)cyclohexyle), salicylate de ci-3-hexenyle, acétate de [(1S)-3-(4-méthylpent-3-ènyl)-1-cyclohex-3-ènyl]méthyle, l'acétate de 3-pentyltetrahydro[2H]pyranyle, propionate de linalyle, acétate de cétyle, acétate de cédryle, acétate d'anisyle, acétate de nopyle, acétate de néryle, acétate de 3a,4,5,6,7,7a-hexahydro-4,7-methanoindèn-6-yle, propanoate de 3a,4,5,6,7,7a-hexahydro-4,7-methano-1H-indèn-6-yl, 3-cyclohexanepropanoate de 2-propényl, 2-hydroxypropane-1,2,3-tricarboxylate de 1,2,3-triéthyle, acétate de (2E)-3,7-diméthylocta-2,6-dièn-1-yle, acétate de 3,5,5-trimethylhexyle, acétate de 3,7-diméthyl-octa-1,6-diene-3-yle, éthanoate de cis-3,7-diméthyl-2,6-octadiènyle, ester de 1-méthyléthyl de l'acide tetradécanoïque, ester 3-méthylbutylique de l'acide 2-hydroxy-benzoïque, ester phénylméthylique de l'acide 2-hydroxy-benzoïque, ester 2-hexylique de l'acide 2-hydroxy-benzoïque, ester méthylique de l'acide 2-hydroxy-Benzoïque, ester éthylique de l'acide acétoacétique, acétate de 3,7-diméthyl-octa-1,6-diène-3-yle, ester 1,2-diéthylique de l'acide 1,2-benzènedicarboxylique, 2-méthylpropanoate de (Z)-hex-3-ènyle, acétate de (4-méthyl-1-propan-2-yl-1-cyclohex-2-enyl), acétate de 3a,4,5,6,7,7a-hexahydro-4,7-méthanoindèn-6-yle, 2,3-époxy-3-phénylbutyrate d'éthyle, 2-aminobenzoate de méthyle, 2-(méthylamino)benzoate de méthyle, benzoate de méthyle, 2,4-dihydroxy-3,6-diméthylbenzoate de méthyle, acétate (3R-(3alpha,3beta,6beta,7beta,8alpha))-octahydro-6-méthoxy-3,6,8,8-tetraméthyl-1H-3a,7-méthanoazulène, salicylate d'hexyle, 4-tert-butylcyclohexyl) acétate, palmitate de méthyle, 1,6-octadieèn-3-ol, 3,7-diméthyl-acétate et le citrate de triéthyle. De manière préférée, des exemples d'esters comprennent le propionate de linalyle, acétate de cétyle, acétate de cédryle, acétate d'anisyle, acétate de nopyle, acétate de néryle, acétate (3R-(3alpha,3beta,6beta,7beta,8alpha))-octahydro-6-méthoxy-3,6,8,8-tetraméthyl-1H-3a,7-méthanoazulène, salicylate d'hexyle, 4-tert-butylcyclohexyl) acétate, palmitate de méthyle, 1,6-Octadien-3-ol, 3,7-dimethyl-acétate, acétate de linalyle et le citrate de triéthyle.

Des exemples d'éthers comprennent, mais ne sont pas limités à, l'éther de benzyle, éther éthylique, ambre gris, oxyde de diphényle, 4,6,6,7,8,8-hexaméthyl-1,3,4,6,7,8-hexahydrocyclopenta[g]isochromene, ambre carane, 1,1-diméthoxy-2,2,5-triméthyl-4-hexène, 3,4,4a,5,8,8a-Hexahydro-3',7-diméthylspiro(1,4-methanonaphthalène-2(1H),2'-oxirane), 2,4,6-Triméthyl-4-phényl-1,3-dioxane, Benzène, 1,1'-Oxybis, Méthyle 2-naphthyl éther, éthyle 2-naphthyl éther, 2,4-Diméthyl-4-phényltetrahydrofurane, (éthoxyméthoxy)cyclododecane, (E)-1-méthoxy-4-(1-propényl)-benzène, 1-méthoxy-4-(2-propenyl)-benzène, Méthyl cédryl éther et l'éther éthylique de 2-naphtyle. De manière préférée, des exemples d'éthers comprennent, le 1,1-diméthoxy-2,2,5-triméthyl-4-hexène, Méthyl cédryl éther et l'éther éthylique de 2-naphtyle.

Des exemples d'alcools comprennent, mais ne sont pas limités à, le menthol ([1R-(1alpha,2beta,5alpha)]-5-méthyl-2-isopropylcyclohexanol), citronellol, géraniol, linalol (par exemple l'éthyle linalol et le tetrahydro linalol), alcool phényléthylique, terpinéol, 2,6-diméthylheptan-2-ol, 2-méthyl-1-phénylpropan-2-ol (diméthyl phényle carbinol), 3-méthyl-5-[2,2,3-triméthylcyclopent-3-èn-1-yl]pent-4-èn-2-ol, 2-phényléthanol, 2-éthyl-4-(2,2,3-triméthyl-1-cyclopent-3-enyl)but-2-èn-1-ol, (E)-4-méthyldec-3-èn-5-ol, alcool cinnamique (3-phényl-2-propèn-1-ol), 3,7-Diméthyl-6-octen-1-ol, p-menth-1-en-8-ol, cis-Hex-3-en-1-ol, 4-Méthyl-3-decen-5-ol, 2,6-diméthyloct-7-en-2-ol, 1,1'-Oxydipropan-2-ol, 3,7-Diméthyl-1,6-nonadien-3-ol, 1,1'-Oxydipropan-2-ol, 2,6-diméthyloct-7-en-2-ol 3,7-Diméthyl octa-1,6-diene-3-ol, 2-éthyl-4-(2,2,3-triméthyl-3-cyclopenten-1-yl)-2-buten-1-ol, phénylméthanol, 4-Méthyl-3-decen-5-ol, 2,6-Diméthyloctan-2-ol, 3,7-Diméthyl-6-octen-1-ol, 4-(1,1-diméthyléthyl)-Cyclohexanol, , (2E)-3,7-diméthyl-2,6-Octadien-1-ol, Hexan-1-ol, exo-1,7,7-Triméthylbicyclo[2.2.1]heptan-2-ol, 2-(2,2,7,7-tetraméthyltricyclo[6.2.1.0 (1,6)]undec-5(4)-en-5-yl)propan-1-ol, 1-Phenyléthanol, 1,1'-Oxydipropan-2-ol, 3,7-Diméthyloctan-3-ol, cis-3,7-Diméthyl-2,6-octadien-1-ol, 3,7-Diméthyl-6-octen-1-ol, Hex-2-en-1-ol, 3,7-Diméthyl octa-1,6-diène-3-ol, 2-Méthyl-4-phénylbutan-2-ol, 2,6-Octadien-1-ol, 3,7-diméthyl-, (2E)-, p-menth-1-en-8-ol, 3-Phénylpropan-1-ol, phénylméthanol, 2,6-diméthyloct-7-èn-2-ol, alpha,beta,2,2,3-pentaméthylcyclopent-3-ène-1-butanol, 3-(5,5,6-triméthylbicyclo[2.2.1]hept-2-yl) cyclohexan-1-ol (IBCH), cis-3-hexèn-1-ol, méthyl-triméthylbicyclo-hexylméthyl-cyclopropyl méthanol alcool benzylique, endo-1,7,7-triméthylbicyclo-[2.2.1]heptan-2-ol, 3,7-diméthyl-6-octèn-1-ol, 3,7-diméthyl-1-octanol, (2E)-3,7-diméthyl-2,6-octadièn-1-ol, cis-3,7-diméthyl-2,6-octadièn-1-ol, 3,7-diméthyl-octa-1,6-diène-3-ol, 2-(4-méthyl-1-cyclohex-3-ènyl)propan-2-ol, 4-méthyl-1-(1-méthyléthyl)-3-cyclohexèn-1-ol, (1R, 2S, 5R)-5-méthyl-2-(1-méthyléthyl)-cyclohexanol, (2E)-3,7-diméthyl-2,6-octadièn-1-ol et le 3-méthylbutan-1-ol. De manière préférée, des exemples d'alcools comprennent l'alpha,beta,2,2,3-pentaméthylcyclopent-3-ène-1-butanol, 3-(5,5,6-triméthylbicyclo[2.2.1]hept-2-yl) cyclohexan-1-ol (IBCH), cis-3-hexenol, méthyl-triméthylbicyclo-hexylméthyl-cyclopropyl méthanol, 3-méthylbutan-1-ol, linalol d'éthyle, tetrahydro linalol et le [1R-(1alpha,2beta,5alpha)]-5-méethyl-2-isopropylcyclohexanol (menthol).

Des exemples d'aldéhydes comprennent, mais ne sont pas limités à, les alcanals linéaires comprenant entre 8 et 18 atomes de carbones, 3,7-Dimethyl-2,6-octadienal (citral), citronellal, aldéhyde de Cyclamen, hydroxycitronellal, 3,7-Diméthyl-2,6-octadienal, Undécanal, alpha-méthyl-4-(1-méthyléthyl)-Benzènepropanal, 3-(4-isopropylphenyl)-2-methylpropanal, 2,4-diméthylcyclohex-3-ene-1-carbaldéhyde, 2,4-diméthylcyclohex-3-ene-1-carbaldéhyde, (2E)-2-Dodecenal, Octanal, Lauryl aldéhyde, Nonanal, (E)-2-Benzylideneoctanal, 2,4-diméthylcyclohex-3-ene-1-carbaldéhyde, 3-(4-éthylphényl)-2,2-diméthylpropanal, 4-Hydroxy-3-methoxybenzaldéhyde , 3-(4-tert-butylphényl)-2-methylpropanal, 3-(4-tert-butylphényl)propanal, 2,6,10-triméthylundec-9-ènal, 4(octahydro-4,7-méthano[5H]indèn-5-ylidène)butanal, 3-(3-propan-2-ylphényl)butanal, 7-hydroxy-3,7-diméthyloctanal (hydroxycitronellal, 3,7-diméthyl-7-hydroxy-octane-1-al), 4-(4-hydroxy-4-méthylpentyl)cyclohex-3-ène-1-carbaldehyde, octahydro-5-méthoxy-4,7-méthano-1H-indène-2-carboxaldehyde, aldéhyde d'alpha-méthyle cinnamique (2-méthyl-3-phényl-2-propènal), 4-méthoxybenzaldehyde (Aldéhyde anisique), aldéhyde en C10 (décanal), undéc-10-enal, aldéhyde en C12 (laurique ou dodécanal), acétaldéhyde de méthyl-nonyle (2-méthylundécanal), aldéhyde en C16, aldéhyde en C6 (hexanal), aldéhyde cinnamique (3-phényl-2-propénal), 3-éthyoxy-4-hydroxybenzaldéhyde (Ethylvanilline), aldéhyde d'hexyl cinnamique (2-benzylidèneheptanal), 3-phénylbutanal (3-phénylbutyraldehyde), 2,4-diméthylcyclohex-3-ène-1-carbaldehyde, 5-heptanal, 2,6-dimethylhept-5-enal, 4-hydroxy-3-méthoxybenzaldehyde (Vanilline), alpha-méthyl-1,3-benzodioxole-5-propionaldehyde, 4-isopropylbenzaldehyde, 3,7-dimethyl-6-octènal, 3,7-diméthyl-2,6-octadiènal, 4-(4-hydroxy-4-méthylpentyl)-3-cyclohexène-1-carboxaldehyde, trans-hex-2-ènal, 2,4,6-triméthyl-3-cyclohexène-1-carboxaldehyde, 2-(4-tert-butylbenzyl)propionaldehyde et le benzaldehyde. De manière préférée, des exemples d'aldéhydes comprennent le 2,4-diméthylcyclohex-3-ène-1-carbaldehyde, 5-heptanal, 2,6-diméthyl-hept-5-enal, 4-hydroxy-3-méthoxybenzaldehyde (Vanilline), alpha-méthyl-1,3-benzodioxole-5-propionaldehyde, citral et le benzaldehyde.

Des exemples de cétones comprennent, mais ne sont pas limités à, les ionones, isométhylionone, cédryle de méthyle, (E)-1-(2,6,6-triméthyl-1-cyclohex-2-enyl)but-2-en-1-one (alpha-damascone), 3-méthyl-2-[(2Z)-pent-2-en-1-yl]cyclopent-2-en-1-one (cis-jasmone), 4-(4-méthoxyphényl)-butan-2-one, 4(3)-(4-méthylpent-3-ènyl)cyclohex-3-ènecarbaldehyde, 1-(1,2,3,4,5,6,7,8-octahydro-2,3,8,8-tetraméthyl-2-naphthalenyl)éthanone, 3-Méthyl-4-(2,6,6-triméthyl-2-cyclohexényl)-3-butèn-2-one, (E)-4-(2,6,6-triméthyl-1-cyclohexen-1-yl)-3-buten-2-one, E)-4-(2,6,6-triméthylcyclohex-2-eneyl)- but-3-en-2-one, 7-Méthyl-2H-benzo-1,5-dioxepin-3(4H)-one, 1-(2,6,6-Triméthyl-1,3-cyclohexadienyl)-2-butèn-1-one, Méthyl hydroxypyrone, 2,2,5-Triméthyl-5-pentylcyclopentan-1-one, 1-(5,5-Diméthyl-1-cyclohexenyl)pent-4-en-1-one, 3-Méthyl-4-(2,6,6-triméthyl-2-cyclohexenyl)-3-buten-2-one, 2-[2-(4-Méthyl-3-cyclohexen-1-yl)propyl]-cyclopentanone, 1-(1,2,3,4,5,6,7,8-octahydro-2,3,8,8-tetraméthyl-2-naphthalenyl)éthanone, (E)-4-(2,6,6-triméthyl-1-cyclohexen-1-yl)-3-buten-2-one, 4-Phenylbutan-2-one 2-Cyclohexyl-1,6-heptadien-3-one, 1-(5,5-Diméthyl-1-cyclohexenyl)pent-4-en-1-one, 2-Buten-1-one, 1-(2,6,6-triméthyl-3-cyclohexen-1-yl)-, 2H-1-Benzopyran-2-one, 1-spiro(4.5)-7-decen-7-yl-4-penten-1-one and 1-spiro(4.5)-6-decen-7-yl-4-penten-1-one, (E)1-(2,6,6-Triméthyl-2-cyclohexen-1-yl)-2-buten-1-one, (E)-4-(2,6,6-triméthyl-1-cyclohexen-1-yl)-3-buten-2-one, Dihydro-5-pentyl-2(3H)-furanone, 2,2,5-Triméthyl-5-pentylcyclopentan-1-one, 1-(1,2,3,4,5,6,7,8-octahydro-2,3,8,8-tetraméthyl-2-naphthalenyl)éthanone, cétone de méthyl cédryl, 7-méthylbenzo[b][1,4]dioxepin-3-one, 1,7,7-triméthylbicyclo[2,2,1]heptan-2-one, 1-benzopyrane-2-one (Coumarine), 1-(2,6,6-triméthyl-1-cyclohex-3-ènyl)but-2-èn-1-one, butan-2,3-dione (Diacétyle), 1-(1,2,3,4,5,6,7,8-octahydro-2,3,8,8,-tetraméthyl-2-naphthyl)éthan-1-one, irones, 1-(2-naphthalényl)éthanone (2-acétonaphtone), menthone, carvone, 3-méthyl-2-pentyl-2-cyclopentènone, 1-(2,6,6-triméthyl-3-cyclohexen-1-yl)-2-butèn-1-one, 1-(2,6,6-triméthyl-2-cyclohexényl)hepta-1,6-dièn-3-one, 2-éthyl-3-hydroxy-4H-pyran-4-one, (5R)-2-méthyl-5-prop-1-èn-2-ylcyclohex-2-èn-1-one, 1-(6-tert-butyl-1,1-diméthyl-2,3-dihydro-1H-indèn-4-yl)éthanone, 1-(5,6,7,8-tetrahydro-3,5,5,6,8,8-hexaméthyl-2-naphthyl)éthan-1-one, 4-(2,6,6-triméthylcyclohex-2-ènyl)-but-3-ène-2-one, octan-2-one, 1-(1,2,3,4,5,6,7,8-Octahydro-2,3,8,8- Tétraméthyl-2-Napthyl)Ethan-1-one et la 1,3,4,6,7,8a-Hexahydro-1,1,5,5-tetramethyl-2H-2,4a-methanonaphthalen-8(5H)-one. De manière préférée, des exemples de cétones comprennent les irones, 1-(2-naphthalényl)éthanone (2-acétonaphtone), menthone, carvone, 3-méthyl-2-pentyl-2-cyclopentènone, 1-(1,2,3,4,5,6,7,8-Octahydro-2,3,8,8- Tétraméthyl-2-Napthyl)Ethan-1-one et la 1,3,4,6,7,8a-Hexahydro-1,1,5,5-tetramethyl-2H-2,4a-methanonaphthalen-8(5H)-one.

Des exemples de lactones comprennent, mais ne sont pas limités à la gamma décalactone (decan-4-olide), Décan-5-olide, Décan-4-olide, Undécan-4-olide gamma undécalactone (undecan-4-olide), cis-jasmone lactone, gamma undecalactone, delta octalacone, delta decalactone et la hexahydro-3,6-diméthyl-2(3H)-benzofuranone. De manière préférée, des exemples de lactones comprennent la gamma undecalactone, delta octalacone, delta decalactone et la hexahydro-3,6-diméthyl-2(3H)-benzofuranone.

Des exemples d'acétals comprennent, mais ne sont pas limités à, le 2,4- diméthyl tetrahydroindenodioxine, diacétal de l'aldéhyde phénylacétique, le glycérylacétal de phénylacetaldéhyde, le diéthyl acétal de citral, le diméthyl acétal de citral, 2,6-octadiènal, 1,1-diméthoxy-2-phényléthane et l'acide 3,7-diméthyl isomérisé. De manière préférée, des exemples d'acétals comprennent le glycérylacétal de phénylacetaldéhyde, le diéthyl acétal de citral, le diméthyl acétal de citral, 2,6-octadiènal et l'acide 3,7-diméthyl isomérisé.

Des exemples de nitriles comprennent, mais ne sont pas limités à, le 3,7-diméthyloct-6-ène nitrile (citronellyle nitrile), tridec-2-ènenitrile, 3-phényl-2-propènenitrile, 3,7-Diméthyl-6-ènenitrile, dodécanenitrile et le 3,7-diméthylnona-2,6-diènenitrile. De manière préférée, des exemples de nitriles comprennent le tridec-2-ènenitrile, 3-phényl-2-propènenitrile, dodécanenitrile et le 3,7-diméthylnona-2,6-diènenitrile.

Des exemples de phénols comprennent, mais ne sont pas limités à, l'eugénol (2-méthoxy-4-(2-propényl)-phénol), iso-eugénol, 5-méthyl-2-(1-méthyléthyl)-phénol, 2-éthoxy-4-méthylphénol, 2-isopropyl-5-methylphenol, 5-méthyl-2-(1-méthyléthyl)-Phénol, 2,6-di-tert-butyl-p-crésol et le 2-éthoxy-4-(méthoxyméthyl)-phénol.

Des exemples d'acides comprennent, mais ne sont pas limités à, l'acide pentanoïque, l'acide butyrique et l'acide 2-méthylpent-2-èn-1-oïque.

Des exemples de terpènes tels que des hydrocarbures terpéniques cycliques (par exemple sesquiterpéniques) ou non cycliques, comprennent, mais ne sont pas limités à, le limonène, 1-méthyl-4-isopropényl-1-cyclohexène, 1-méthyl-4-isopropyl-1,4-cyclohexadiène, 7-méthyl-3-méthylèneocta-1,6-diène, 1-méthyl-4-(1-méthyléthyl)-1,3-cyclohexadiène, 2,6,6-triméthylbicyclo[3.1.1]hept-2-ène, 6,6-diméthyl-2-méthylènebicyclo[3.1.1]heptane, 2,2-diméthyl-3-méthylènebicyclo-[2.2.1]-heptane, (3R-(3alpha,3abeta,6beta,7beta,8aalpha))-Octahydro-6-méthoxy-3,6,8,8-tetraméthyl-1H-3a,7-méthanoazulène, 4,11,11-triméthyl-8-méthylene-(1R,4E,9S)-Bicyclo[7.2.0.]undec-4-ene, [1R,(1R*,4E,9S*)]-4,11,11-triméthyl-8-méthylène-bicyclo[7.2.0]undec-4-ène, 1-méthyl-4-(1-méthyléthyl)benzène et les huiles essentielles à sesquiterpènes. De manière préférée, des exemples de terpènes comprennent des huiles essentielles à sesquiterpènes.

Des exemples de composés hétérocycliques azotés ou soufrés, saturés ou insaturés, comprennent, mais ne sont pas limités à, l'indole, 1,3-benzopyrrole, tétrahydro-4-méthyl-2-(2-méthyl-1-propényl)-2H-pyrane, 2-méthyl-pyrazine, 4-méthyl-5-hydroxyéthyl thiazole (2-(4-méthylthiazol-5-yl)éthanol), 6-tert-butylquinoline, 6-(isopropyl)quinoline, (3aR-(3aalpha,5abeta,9aalpha,9bbeta))-Dodécahydro-3a,6,6,9a-tetraméthylnaphtho(2,1-b)furane, 2-Isopropyl-4-méthyl-1,3-thiazole, cis-2-méthyl-4-propyl-1,3-oxathiane, 6(8)-(1-Methylpropyl)Quinoléine et les pyrazines. De manière préférée, des exemples de composés hétérocycliques azotés ou soufrés, saturés ou insaturés, comprennent la 6-tert-butylquinoline, 6-(isopropyl)quinoline, cis-2-méthyl-4-propyl-1,3-oxathiane, 6(8)-(1-Methylpropyl)Quinoléine et les pyrazines.

Des exemples de produits complexes d'origine naturelle comprennent, mais ne sont pas limités à, des huiles essentielles extraites à partir des différentes parties de plantes (fleurs, tiges, feuilles, fruits, écorces, racines, parties boisées, herbes, aiguilles, sève et gommes), des résinoïdes, des concrètes, ou absolues obtenues à partir de ces derniers. De manière préférée, des exemples de produits complexes d'origine naturelle comprennent de l'huile d'armoise herbe blanche (Artemisia Herba-Alba), Huile de feuille de Pogostemon Cablin (Pogostemon Cablin Leaf Oil), Huile d'écorce de Citrus nobilis (Citrus nobilis peel oil), Huile de feuille de Barosma Betulina (Barosma Betulina Leaf Extract), extrait de l'écorce de citronnier (Citrus Limon Peel extract), de l'huile de feuille d'Eucalyptus globulus, huile de Dipterocarpus turbinatus (Dipterocarpus turbinatus Balsam Oil), huile de feuille de Pogostemon cablin, huile de feuille de Rosmarinus officinalis, huile de Juniperus virginiana, huile de feuille de menthe des champs (Mentha Arvensis), huile de feuille de menthe verte (Mentha viridis), huile d'écorce de Citrus Aurantium Dulcis, extrait d'écorce de Citrus Aurantium Dulcis, huile de feuille de Cyprès méditerranéen (Cupressus sempervirens), huile de feuille de patchouli (Pogostemon cablin), huile de Cèdre Texas (Juniperus Mexicana) et l'huile de Lavandula Hybride (Lavandula Hybrida).

La composition aqueuse parfumée peut éventuellement comprendre un ou plusieurs ingrédients supplémentaires, choisis parmi les solvants, les diluants, les additifs, les excipients, etc. Les matières premières et les quantités appropriées sont bien connues de l'homme du métier.

Selon un mode de réalisation particulier, la composition aqueuse parfumée selon l'invention comprend en outre au moins un additif choisi parmi les agents anti-mousse, agents antioxydants, agents chélatants, filtres UV, conservateurs, agents épaississants, ingrédients actifs cosmétiques, agents hydratants, humectants, adoucissants, pigments, colorants, agents réfrigérants, agents ajusteurs de pH, agents bactéricides, agents bactériostatiques, insecticides, agents répulsifs, paillettes, actifs, et leurs mélanges. Préférentiellement, la quantité d'eau ajoutée est déduite de la quantité de ces additifs de sorte que la somme de l'eau et des éventuels additifs est comprise dans la gamme de la quantité d'eau décrite ci-dessus.

Les modes de réalisation détaillés ci-dessus peuvent être aisément combinés les uns avec les autres de façon non limitative.

### EXEMPLES

### Exemple 1 : parfum No. 1

Les composants décrits dans le tableau 1 sont mélangés afin d'obtenir le parfum No. 1.

| **parfum 1** | |
|---|---|
| **Matières premières** | **Dosages (%)** |
| Huile Essentielle (HE) d'orange | 60.00 |
| HE de bergamote | 20.00 |
| HE de petitgrain | 10.00 |
| HE de citron | 4.00 |
| Citrate d'éthyle | 2.80 |
| HE de mandarine | 2.00 |
| HE d'ylang | 1.00 |
| HE de néroli | 0.20 |

### Exemple 2 : Composition aqueuse parfumée N°1

Les composants décrits dans le tableau 2 sont mélangés afin d'obtenir la composition aqueuse parfumée N°1.

| **Composition aqueuse parfumée 1** | |
|---|---|
| **Matière premières** | **Dosages (%)** |
| Parfum 1 | 5.10 |
| Monopropylène glycol | 3.90 |
| caprylyl glycol (A-leen 8) | 1.00 |
| Polyglyceryl-6 Caprylate/Caprate et Sodium Caproyl/Lauroyl Lactylate (Easytens S2) | 20.00 |
| Eau | 69.00 |
| Conservateur | 1.00 |
| Résultats à J+1 | Clair |

La composition aqueuse parfumée selon l'invention est transparente et homogène.

La composition aqueuse parfumée 1 est formulée à partir d'un complexe parfumant comprenant : 51 % du parfum N°1, 10 % de caprylyl glycol et 39% de mono propylène glycol. Les pourcentages étant exprimés en poids par rapport au poids total des 3 composants ci-dessus. Puis, 10% du complexe parfumant est mélangé à 20% d'un mélange de Polyglyceryl-6 Caprylate/Caprate et Sodium Caproyl/Lauroyl Lactylate préférentiellement chauffé. Enfin, 69% d'eau est ajouté au mélange pour obtenir la composition aqueuse parfumée 1. Les pourcentages étant exprimés en poids par rapport au poids total de la composition aqueuse parfumée.

Le conservateur utilisé est un conservateur respectant la charte Cosmos en vigueur en 2022, soluble dans l'eau et transparent. Le conservateur choisit est le Epsan C.

### Exemple 3 : parfum No. 2

Les composants décrits dans le tableau 3 sont mélangés afin d'obtenir le parfum No. 2.

| **parfum 2** | |
|---|---|
| **Matières premières** | **Dosages (%)** |
| HE de citron | 44.94 |
| Terpènes d'orange | 33.71 |
| Citrate d'éthyle | 1.18 |
| Acétate de linalyle | 8.43 |
| HE de sauge sclarée | 2.25 |
| Citral | 2.25 |
| HE de cardamome | 1.35 |
| Caproate d'éthyle 10% sol. citrate d'éthyle | 1.12 |
| HE de cèdre | 1.12 |
| HE de poivre | 0.90 |
| HE de gingembre | 0.90 |
| HE de carvi | 0.56 |
| Extrait CO2 de gingembre | 0.45 |
| HE d'eucalyptus | 0.34 |
| HE de menthe poivrée | 0.22 |
| Melonal | 0.22 |
| HE de lentisque | 0.06 |

### Exemple 4 : Compositions aqueuses parfumées N°2 à 4

Les composants décrits dans le tableau 4 sont mélangés afin d'obtenir les compositions aqueuses parfumées N°2 à 4.

| **Compositions aqueuses parfumées** | **2** | **3** | **4** |
|---|---|---|---|
| **Matières premières** | **Dosage (%)** | | |
| Parfum 2 | 8.10 | 8.10 | 7.00 |
| Monopropylène glycol | 1.90 | 0.90 | - |
| caprylyl glycol (A-leen 8) | - | 1.00 | 3.00 |
| Polyglyceryl-6 Caprylate/Caprate et Sodium Caproyl/Lauroyl Lactylate (Easytens S2) | 20.00 | 20.00 | 20.00 |
| Eau | 69.00 | 69.00 | 69.00 |
| Conservateur | 1.00 | 1.00 | 1.00 |
| Résultats à J+1 | Déphase | Déphase | Clair |

Les compositions aqueuses parfumées 2, 3, 4 sont formulées à partir de complexes parfumants comprenant :
81 % ou 70% du parfum N°2, 0 ou 10 % ou 30% de caprylyl glycol et 0 ou 9 ou 19 % de mono propylène glycol. Les pourcentages étant exprimés en poids par rapport au poids total des 3 composants ci-dessus. Puis, 10% du complexe parfumant est mélangé à 20% d'un mélange de Polyglyceryl-6 Caprylate/Caprate et Sodium Caproyl/Lauroyl Lactylate préférentiellement chauffé. Enfin, 69% d'eau est ajouté au mélange pour obtenir les compositions aqueuses parfumées 2, 3, 4. Les pourcentages étant exprimés en poids par rapport au poids total de la composition aqueuse parfumée. Le conservateur utilisé est un conservateur respectant la charte Cosmos vigueur en 2022 en soluble dans l'eau et transparent. Le conservateur choisit est le Epsan C.

Il ressort de ces différentes compositions aqueuses parfumées que le solvant caprylyl glycol (A-leen 8) participe à obtenir une composition transparente et homogène.

La composition aqueuse parfumée 4 est réalisée avec la quantité la plus élevée de parfum dans le complexe parfumant à savoir 70% en poids du poids total du complexe parfumant, c'est-à-dire 7% en poids du poids total de la composition aqueuse parfumée finale numéro 4.

Cette composition aqueuse parfumée est réalisée avec la quantité la plus élevée de A-leen 8 dans le complexe parfumant à savoir 30% en poids du poids total du complexe parfumant, c'est-à-dire 3% en poids du poids total de la composition aqueuse parfumée finale numéro 4.

Cette composition aqueuse parfumée est réalisée avec la quantité la plus basse de Monopropylène glycol dans le complexe parfumant à savoir 0 % en poids du poids total du complexe parfumant, c'est-à-dire 0 % en poids du poids total de la composition aqueuse parfumée finale numéro 4.

La composition aqueuse parfumée 4 selon l'invention est transparente et homogène.

### Exemple 5 : parfum N° 3

Les composants décrits dans le tableau 5 sont mélangés afin d'obtenir le parfum N° 3.

| **parfum 3** | |
|---|---|
| **Matière premières** | **Dosages (%)** |
| Linalol | 16.13 |
| Geraniol | 16.13 |
| Acétate de linalyl | 16.13 |
| Citronellol | 11.29 |
| HE d'orange | 8.06 |
| HE de citron | 8.06 |
| Terpineol alpha | 8.06 |
| HE de mandarine | 1.61 |
| Gamma undecalactone | 1.61 |
| Salicylate de benzyl | 1.61 |
| Citrate d'éthyle | 1.52 |
| Acétate cis-3-hexenyle | 1.29 |
| HE de palmarosa | 1.13 |
| HE de bucchu | 0.97 |
| HE de géranium | 0.97 |
| Gamma decalactone | 0.81 |
| Acétate d'iso amyle | 0.81 |
| Acétate de benzyle | 0.81 |
| Alcool phenyléthylique | 0.81 |
| lonone beta | 0.81 |
| Acétate d'éthyle | 0.48 |
| HE de cardamome | 0.16 |
| Coumarine | 0.16 |
| Raspberry ketone | 0.16 |
| Veltol | 0.16 |
| Extrait CO2 de cassis | 0.10 |
| Anthranylate de méthyle | 0.08 |
| Hexanal | 0.08 |

### Exemple 6 : Composition aqueuse parfumée N°5

Les composants décrits dans le tableau 6 sont mélangés afin d'obtenir la composition aqueuse parfumée N°5.

| **Composition aqueuse parfumée 5** | |
|---|---|
| **Matière premières** | **Dosages (%)** |
| Parfum 3 | 0.50 |
| Monopropylène glycol | 8.00 |
| caprylyl glycol (A-leen 8) | 1.50 |
| Polyglyceryl-6 Caprylate/Caprate et Sodium Caproyl/Lauroyl Lactylate (Easytens S2) | 20.00 |
| Eau | 69.00 |
| Conservateur | 1.00 |
| Résultat à J+1 | Clair |

La composition aqueuse parfumée 5 est formulée à partir d'un complexe parfumant comprenant : 5 % du parfum N°3, 15 % de caprylyl glycol et 80% de mono propylène glycol. Les pourcentages étant exprimés en poids par rapport au poids total des 3 composants ci-dessus. Puis, 10% du complexe parfumant est mélangé à 20% d'un mélange de Polyglyceryl-6 Caprylate/Caprate et Sodium Caproyl/Lauroyl Lactylate préférentiellement chauffé. Enfin, 69% d'eau est ajouté au mélange pour obtenir la composition aqueuse parfumée N°5. Les pourcentages étant exprimés en poids par rapport au poids total de la composition aqueuse parfumée. Le conservateur utilisé est un conservateur respectant la charte Cosmos vigueur en 2022 en soluble dans l'eau et transparent. Le conservateur choisit est le Epsan C.

Cette composition parfumante aqueuse est réalisée avec la quantité la plus basse de parfum dans le complexe parfumant à savoir 5% en poids du poids total du complexe parfumant c'est-à-dire 0,5% en poids du poids total de la composition aqueuse parfumée finale numéro 5.

La composition aqueuse parfumée selon l'invention est transparente et homogène.

### Exemple 7 : parfum N° 4

Les composants décrits dans le tableau 7 sont mélangés afin d'obtenir le parfum N° 4.

| **Parfum 4** | |
|---|---|
| **Matière premières** | **Dosages (%)** |
| HE de bergamote | 21.71 |
| Linalol | 15.50 |
| Acétate de benzyle | 12.40 |
| HE d'orange | 9.30 |
| Citronellol | 7.75 |
| Terpineol alpha | 6.20 |
| HE de mandarine | 6.20 |
| Alcool phenyléthylique | 2.48 |
| HE de palmarosa | 2.48 |
| Indol 10% sol. citrate d'éthyle | 1.55 |
| Acétate cis-3-hexenyle | 1.55 |
| HE de lavande | 1.55 |
| HE de davana | 1.24 |
| HE de petitgrain | 0.93 |
| Gamma undecalactone | 0.93 |
| Anthranylate de méthyle | 0.93 |
| HE de cèdre | 0.93 |
| Acétate de phenylethyle | 0.93 |
| HE d'ylang | 0.93 |
| Coumarine 10% sol. citrate d'éthyle | 0.62 |
| Raspberry ketone | 0.62 |
| Vanilline | 0.62 |
| HE de néroli 10% sol. citrate d'éthyle | 0.62 |
| lonone beta | 0.31 |
| Cis-3-hexenol | 0.31 |
| Eugenol | 0.31 |
| HE cardamome | 0.31 |
| Veltol | 0.31 |
| Gamma decalactone | 0.16 |
| Citrate d'éthyle | 0.16 |
| Acetyl methyl carbinol 10% sol. citrate d'éthyle | 0.16 |

### Exemple 8 : Composition aqueuse parfumée N°6

Les composants décrits dans le tableau 8 sont mélangés afin d'obtenir la composition aqueuse parfumée N°6.

| **Composition aqueuse parfumée 6** | |
|---|---|
| **Matière premières** | **Dosages (%)** |
| Parfum 4 | 0.50 |
| Monopropylène glycol | 8.50 |
| caprylyl glycol (A-leen 8) | 1.00 |
| Polyglyceryl-6 Caprylate/Caprate et Sodium Caproyl/Lauroyl Lactylate (Easytens S2) | 20.00 |
| Eau | 69.00 |
| Conservateur | 1.00 |
| Résultat à J+1 | Clair |

La composition aqueuse parfumée 6 est formulée à partir d'un complexe parfumant comprenant : 5 % du parfum N°4, 10 % de caprylyl glycol et 85% de mono propylène glycol. Les pourcentages étant exprimés en poids par rapport au poids total des 3 composants ci-dessus. Puis, 10% du complexe parfumant est mélangé à 20% d'un mélange de Polyglyceryl-6 Caprylate/Caprate et Sodium Caproyl/Lauroyl Lactylate préférentiellement chauffé. Enfin, 69% d'eau est ajouté au mélange pour obtenir la composition aqueuse parfumée 6. Les pourcentages étant exprimés en poids par rapport au poids total de la composition aqueuse parfumée. Le conservateur utilisé est un conservateur respectant la charte Cosmos vigueur en 2022 en soluble dans l'eau et transparent. Le conservateur choisit est le Epsan C.

Cette composition aqueuse parfumée est réalisée avec la quantité la plus basse de A-leen 8 dans le complexe parfumant à savoir 10% en poids du poids total du complexe parfumant, c'est-à-dire 1% en poids du poids total de la composition aqueuse parfumée finale numéro 6.

Cette composition aqueuse parfumée est réalisée avec la quantité la plus élevée de monopropylène glycol dans le complexe parfumant à savoir 85% en poids du poids total du complexe parfumant, c'est-à-dire 8,5% en poids du poids total de la composition aqueuse parfumée finale numéro 6.

La composition aqueuse parfumée selon l'invention est transparente et homogène.

### Exemple 9 : Composition aqueuse parfumée N°7

Les composants décrits dans le tableau 9 sont mélangés afin d'obtenir la composition aqueuse parfumée N°7.

| **Composition aqueuse parfumée 7** | |
|---|---|
| **Matière premières** | **Dosages (%)** |
| Parfum 1 | 7.10 |
| Monopropylène glycol | 3.70 |
| caprylyl glycol (A-leen 8) | 1.20 |
| Polyglyceryl-6 Caprylate/Caprate et Sodium Caproyl/Lauroyl Lactylate (Easytens S2) | 18.00 |
| Eau | 69.00 |
| Conservateur | 1.00 |
| Résultat à J+1 | Trouble |

La composition aqueuse parfumée 7 est formulée à partir d'un complexe parfumant comprenant : 59.17% du parfum N°1, 10% de caprylyl glycol et 30.83% de mono propylène glycol. Puis, 12% du complexe parfumant est mélangé à 18% d'un mélange de Polyglyceryl-6 Caprylate/Caprate et Sodium Caproyl/Lauroyl Lactylate préférentiellement chauffé. Enfin, 69% d'eau est ajouté au mélange pour obtenir la composition aqueuse parfumée 7.

Le conservateur utilisé est un conservateur respectant la charte Cosmos vigueur en 2022 en soluble dans l'eau et transparent. Le conservateur choisit est le Epsan C.

Cette composition aqueuse parfumée est réalisée avec une quantité de solubilisant Easytens S2 inférieure à notre sélection, c'est-à-dire 18% en poids du poids total de la composition aqueuse parfumée finale numéro 7.

La composition aqueuse parfumée n'est pas transparente.

### Exemple 10 : Composition aqueuse parfumée N°8

Les composants décrits dans le tableau 10 sont mélangés afin d'obtenir la composition aqueuse parfumée N°8.

| **Composition aqueuse parfumée 8** | |
|---|---|
| **Matière premières** | **Dosages (%)** |
| Parfum 1 | 7.10 |
| Monopropylène glycol | 2.90 |
| caprylyl glycol (A-leen 8) | 2.00 |
| Polyglyceryl-6 Caprylate/Caprate et Sodium Caproyl/Lauroyl Lactylate (Easytens S2) | 18.00 |
| Eau | 69.00 |
| Conservateur | 1.00 |
| Résultat à J+1 | Trouble |

La composition aqueuse parfumée 8 est formulée à partir d'un complexe parfumant comprenant: 59.17% du parfum N°1, 16.67% de caprylyl glycol et 24.16% de mono propylène glycol. Puis, 12% du complexe parfumant est mélangé à 18% d'un mélange de Polyglyceryl-6 Caprylate/Caprate et Sodium Caproyl/Lauroyl Lactylate préférentiellement chauffé. Enfin, 69% d'eau est ajouté au mélange pour obtenir la composition aqueuse parfumée 8.

Le conservateur utilisé est un conservateur respectant la charte Cosmos vigueur en 2022 en soluble dans l'eau et transparent. Le conservateur choisit est le Epsan C.

Cette composition aqueuse parfumée est réalisée avec une quantité de solubilisant Easytens S2 inférieure à notre sélection, c'est-à-dire 18% en poids du poids total de la composition aqueuse parfumée finale numéro 8.

La composition aqueuse parfumée n'est pas transparente.

L'invention n'est pas limitée aux modes de réalisations précédemment décrits et s'étend à tous les modes de réalisation couverts et les utilisations telles que spécifiées par les revendications.

## Revendications

1. Composition parfumante comprenant les composants suivants :
- de 25 à 34% d'un complexe parfumant comprenant les composants suivants : - 5 à 70% d'un parfum, - 10 à 30% de caprylyl glycol comme solvant, - 0 à 85% de mono propylène glycol comme solvant, les pourcentages étant exprimés en poids par rapport au poids total des 3 composants ci-dessus, et
- de 66 à 75% d'un mélange de Polyglyceryl-6 Caprylate/Caprate et Sodium Caproyl/Lauroyl Lactylate,
les pourcentages étant exprimés en poids par rapport au poids total des 2 composants ci-dessus.

2. Composition parfumante selon la revendication 1 dans laquelle le complexe parfumant ne comprend pas d'eau.

3. Composition aqueuse parfumée comprenant les composants suivants :
- de 20 à 30% de la composition parfumante selon la revendication 1 ou 2,
- de 70 à 80% d'eau,
les pourcentages étant exprimés en poids par rapport au poids total des 2 composants ci-dessus.

4. Composition aqueuse parfumée selon la revendication précédente sous forme de micro-émulsion.

5. Composition aqueuse parfumée selon l'une quelconque des deux revendications précédentes ou composition parfumante selon la revendication 1 ou 2 étant transparente.

6. Composition aqueuse parfumée selon l'une quelconque des trois revendications précédentes ou composition parfumante selon la revendication 1 ou 2 ne comprenant pas de solvant éthoxylé.

7. Composition aqueuse parfumée selon l'une quelconque des quatre revendications précédentes ou composition parfumante selon la revendication 1 ou 2 étant d'origine naturelle.

8. Procédé de préparation d'une composition parfumante selon l'une quelconque des revendications 1 ou 2 comprenant :
- la préparation du complexe parfumant,
- le chauffage du mélange de Polyglyceryl-6 Caprylate/Caprate et Sodium Caproyl/Lauroyl Lactylate,
- l'ajout du mélange de Polyglyceryl-6 Caprylate/Caprate et Sodium Caproyl/Lauroyl Lactylate chauffé au complexe parfumant,
- l'homogénéisation de la composition parfumante obtenue.

9. Procédé de préparation d'une composition aqueuse parfumée selon l'une quelconque des revendications 3 à 7 comprenant :
- l'ajout d'eau à la composition parfumante selon la revendication 1 ou 2,
- l'homogénéisation de la composition aqueuse parfumée obtenue.

10. Procédé de préparation selon la revendication précédente comprenant une étape ultérieure d'ajout d'un conservateur, en quantité comprise entre 0.8% et 1.2%, en poids du poids total de la composition.

11. Procédé de préparation selon l'une quelconque des deux revendications précédentes comprenant éventuellement une étape ultérieure de macération, une étape de glaçage, puis une étape de filtration.

12. Utilisation de la composition aqueuse parfumée selon l'une quelconque des revendications 3 à 7 ou de la composition parfumante selon la revendication 1 ou 2 comme tout ou partie d'une base cosmétique ou d'un produit de parfumerie fine ou d'une base détergente ou d'un parfum d'ambiance.

13. Base cosmétique, base détergente ou parfum d'ambiance comprenant la composition aqueuse parfumée selon l'une quelconque des revendications 3 à 7 ou la composition parfumante selon la revendication 1 ou 2.

14. Utilisation selon la revendication 12 pour masquer les mauvaises odeurs.

## Patentansprüche

1. Duftzusammensetzung, die die folgenden Bestandteile umfasst:
- 25 bis 34 % eines Duftkomplexes, der die folgenden Komponenten umfasst: - 5 bis 70 % eines Parfüms, - 10 bis 30 % Caprylylglykol als Lösungsmittel, - 0 bis 85 % Monopropylenglykol als Lösungsmittel, wobei die Prozentsätze als Gewichtsanteile bezogen auf das Gesamtgewicht der 3 oben genannten Komponenten ausgedrückt werden, und
- 66 bis 75 % einer Mischung aus Polyglyceryl-6-caprylat/caprat und Natriumcaproyl/-lauroyllactylat, wobei die Prozentsätze als Gewichtsanteile bezogen auf das Gesamtgewicht der 2 oben genannten Komponenten ausgedrückt werden.

2. Duftzusammensetzung nach Anspruch 1, wobei der Duftkomplex kein Wasser umfasst.

3. Wässrige Duftzusammensetzung, die die folgenden Bestandteile umfasst:
- 20 bis 30 % der Duftzusammensetzung nach Anspruch 1 oder 2,
- 70 bis 80 % Wasser,
wobei die Prozentsätze als Gewichtsanteile bezogen auf das Gesamtgewicht der 2 oben genannten Komponenten ausgedrückt werden.

4. Wässrige Duftzusammensetzung nach dem vorhergehenden Anspruch in Form einer Mikroemulsion.

5. Wässrige Duftzusammensetzung nach einem der beiden vorhergehenden Ansprüche oder Duftzusammensetzung nach Anspruch 1 oder 2, die transparent ist.

6. Wässrige Duftzusammensetzung nach einem der drei vorhergehenden Ansprüche oder Duftzusammensetzung nach Anspruch 1 oder 2, die kein ethoxyliertes Lösungsmittel umfasst.

7. Wässrige Duftzusammensetzung nach einem der vier vorhergehenden Ansprüche oder Duftzusammensetzung nach Anspruch 1 oder 2, die natürlichen Ursprungs ist.

8. Verfahren zur Herstellung einer Duftzusammensetzung nach einem der Ansprüche 1 oder 2, umfassend:
- Herstellen des Duftkomplexes,
- Erhitzen der Mischung aus Polyglyceryl-6-caprylat/caprat und Natriumcaproyl/-lauroyllactylat,
- Hinzufügen der erwärmten Mischung aus Polyglyceryl-6-caprylat/caprat und Natriumcaproyl/-lauroyllactylat zum Duftkomplex,
- Homogenisieren der erhaltenen Duftzusammensetzung.

9. Verfahren zur Herstellung einer wässrigen Duftzusammensetzung nach einem der Ansprüche 3 bis 7, umfassend:
- Hinzufügen von Wasser zur Duftzusammensetzung nach Anspruch 1 oder 2,
- Homogenisieren der erhaltenen wässrigen Duftzusammensetzung.

10. Herstellungsverfahren nach dem vorhergehenden Anspruch, umfassend einen nachfolgenden Schritt des Hinzufügens eines Konservierungsmittels in einer Menge zwischen 0,8 % und 1,2 %, bezogen auf das Gesamtgewicht der Zusammensetzung.

11. Herstellungsverfahren nach einem der beiden vorhergehenden Ansprüche, gegebenenfalls umfassend einen nachfolgenden Mazerationsschritt, einen Kühlschritt und anschließend einen Filtrationsschritt.

12. Verwendung der wässrigen Duftzusammensetzung nach einem der Ansprüche 3 bis 7 oder der Duftzusammensetzung nach Anspruch 1 oder 2 als Ganzes oder Teil einer Kosmetikproduktbasis oder eines Feinparfümerieprodukts oder einer Reinigungsmittelbasis oder eines Raumdufts.

13. Kosmetikproduktbasis, Reinigungsmittelbasis oder Raumduft, umfassend die wässrige Duftzusammensetzung nach einem der Ansprüche 3 bis 7 oder die Duftzusammensetzung nach Anspruch 1 oder 2.

14. Verwendung nach Anspruch 12 zum Überdecken von unangenehmen Gerüchen.

## Claims

1. Fragrancing composition comprising the following components:
- from 25 to 34% fragrancing complex comprising the following components: - 5 to 70% fragrance, - 10 to 30% caprylyl glycol as a solvent, - 0 to 85% monopropylene glycol as a solvent, the percentages being expressed by weight relative to the total weight of the 3 components above, and
- from 66 to 75% a mixture of Polyglyceryl-6 Caprylate/Caprate and Sodium Caproyl/Lauroyl Lactylate, the percentages being expressed by weight relative to the total weight of the 2 components above.

2. Fragrancing composition according to claim 1, wherein the fragrancing complex does not comprise water.

3. Fragranced aqueous composition comprising the following components:
- 20 to 30% the fragrancing composition according to claim 1 or 2,
- 70 to 80% water,
the percentages being expressed by weight relative to the total weight of the 2 components above.

4. Fragranced aqueous composition according to the preceding claim in the form of a micro-emulsion.

5. Fragranced aqueous composition according to any one of the preceding two claims or fragrancing composition according to claim 1 or 2 being transparent.

6. Fragranced aqueous composition according to any one of the preceding three claims or fragrancing composition according to claim 1 or 2 comprising no ethoxylated solvent.

7. Fragranced aqueous composition according to any one of the preceding four claims or fragrancing composition according to claim 1 or 2 being of natural origin.

8. Method for preparing a fragrancing composition according to either one of claims 1 or 2 comprising:
- preparing the fragrancing complex,
- heating the mixture of Polyglyceryl-6 Caprylate/Caprate and Sodium Caproyl/Lauroyl Lactylate,
- adding the heated mixture of Polyglyceryl-6 Caprylate/Caprate and Sodium Caproyl/Lauroyl Lactylate to the fragrancing complex,
- homogenising the obtained fragrancing composition.

9. Method for preparing a fragranced aqueous composition according to any one of claims 3 to 7 comprising:
- adding water to the fragrancing composition according to claim 1 or 2,
- homogenising the obtained fragranced aqueous composition.

10. Preparation method according to the preceding claim, comprising a subsequent step of adding a preservative, in an amount between 0.8% and 1.2%, by weight of the total weight of the composition.

11. Preparation method according to either one of the preceding two claims, optionally comprising a subsequent maceration step, a glazing step, and then a filtration step.

12. Use of the fragranced aqueous composition according to any one of claims 3 to 7 or the fragrancing composition according to claim 1 or 2 as all or part of a cosmetic base or of a fine fragrance product or of a detergent base or of a room fragrance.

13. Cosmetic base, detergent base or room fragrance comprising the fragranced aqueous composition according to any one of claims 3 to 7 or the fragrancing composition according to claim 1 or 2.

14. Use according to claim 12 for masking bad odours.
